# EUROPÄISCHE PATENTSCHRIFT

(11) **EP 1 828 082 B1**
(45) Veröffentlichungstag und Bekanntmachung des Hinweises auf die Patenterteilung: **23.04.2008**
(21) Anmeldenummer: 05817964.9
(22) Anmeldetag: 08.12.2005
(51) Int. Cl.: C07C 11/24, C07D 207/26

(54) **VERFAHREN ZUR REINIGUNG EINES APPARATES VON ABLAGERUNGEN AUS EINEM VERFAHREN ZUR RÜCKGEWINNUNG VON NMP DURCH EINLEITEN VON HEISSEM WASSER UND RÜHREN**
METHOD FOR CLEANING DEPOSITS FROM EQUIPMENT THAT HAVE ACCUMULATED DURING A METHOD FOR RECOVERING NMP BY THE INTRODUCTION OF HOT WATER AND AGITATION
PROCEDE POUR NETTOYER UN APPAREIL DES DEPOTS RESULTANT D'UN PROCEDE VISANT A RECUPERER UNE NMP PAR INTRODUCTION ET BRASSAGE D'EAU CHAUDE

(30) Priorität: 16.12.2004 DE 102004060654
(43) Veröffentlichungstag der Anmeldung: 05.09.2007
(73) Patentinhaber: BASF SE, 67056 Ludwigshafen (DE)
(72) Erfinder: ALEMANY, Aurelie, 68159 Mannheim (DE); VICARI, Maximilian, 67117 Limburgerhof (DE); KIRCHNER, Tanja, 55283 Nierstein (DE); STEGLICH, Frank, 67269 Grünstadt (DE)
(74) Vertreter: Hörschler, Wolfram Johannes
(86) Internationale Anmeldenummer: PCT/EP2005/013168
(87) Internationale Veröffentlichungsnummer: WO 2006/063727

(56) Entgegenhaltungen:
- WO-A-03/053924
- DE-A1- 1 934 304
- PÄSSLER ET AL: "ULLMANN'S ENCYCLOPEDIA OF INDUSTRIAL CHEMISTRY" [Online] 15. Juni 2000 (2000-06-15), WILEY INTERSCIENCE , XP002373222 Gefunden im Internet: URL:http://www.mrw.interscience.wiley.com/ ueic/articles/a01_097/sect4-fs.html> [gefunden am 2006-03-21] Production 4.2.1. BASF Process (Sachsse-Bartholome)
- HAASE ET AL: "ULLMANN'S ENCYCLOPEDIA OF INDUSTRIAL CHEMISTRY" [Online] 15. Juni 2000 (2000-06-15), WILEY INTERSCIENCE , XP002373223 Gefunden im Internet: URL:http://www.mrw.interscience.wiley.com/ ueic/articles/a16_403/sect2-fs.html> [gefunden am 2006-03-21] Metals, Surface Treatment 2. Cleaning
- HARREUS ET AL: "ULLMANN'S ENCYCLOPEDIA OF INDUSTRIAL CHEMISTRY" [Online] 15. Juni 2000 (2000-06-15), , XP002373224 Gefunden im Internet: URL:http://www.mrw.interscience.wiley.com/ ueic/articles/a22_457/sect3-fs.html> [gefunden am 2006-03-21] 2.Pyrrolidone 3. N-Methyl-2-pyrrolidone

## Beschreibung

Die Erfindung betrifft ein Verfahren zur Reinigung eines Apparates von den Ablagerungen aus einem Verfahren zur Rückgewinnung von gereinigtem NMP.

Acetylen wird großtechnisch überwiegend durch Partialoxidation von Kohlenwasserstoffen mit Sauerstoff in einer Hochtemperaturreaktion hergestellt. Die Herstellung von Acetylen durch Partialoxidation von Kohlenwasserstoffen ist als BASF-Verfahren (Sachsse-Bartholome) bekannt und beispielsweise in Ullmann's Encyclopedia of Industrial Chemistry, Sixth Edition, 2000 Electronic Release, Pages 1 to 5, beschrieben.

Aus dem Reaktionsgemisch der Partialoxidation wird Acetylen überwiegend durch selektive Absorption mit einem Lösungsmittel, insbesondere N-Metylpyrrolidon, im Folgenden abgekürzt als NMP bezeichnet, Methanol, Ammoniak oder Dimethylformamid, gewonnen. Nach dem Ausgasen des Acetylens aus dem beladenen Lösungsmittel verbleibt ein Lösungsmittelstrom, der Oligo- oder Polymere des Acetylens als Verunreinigungen enthält, üblicherweise in einem Anteil von etwa 0,1 Gew.-%. In der zitierten Literaturstelle in Ullmann's wird beschrieben, dass im Verfahren zur extraktiven Reinigung von Acetylen mit NMP zwecks Minimierung der Beladung des NMP mit Polymeren des Acetylens, etwa 2 % des NMP-Stromes kontinuierlich aus dem Prozess abgezogen und unter Vakuum destilliert wird, wobei die Polymere als praktisch trockener Rückstand zurückbleiben, der entsorgt wird. Dabei wird gereinigtes NMP gewonnen, das in die extraktive Reinigung recycliert wird.

Das mit etwa 0,1 Gew.-% Polymer verunreinigte NMP wird bei bestehenden Verfahren zunächst in einer Vorverdampfung unter Vakuum aufkonzentriert, zum Beispiel auf einen Polymergehalt von etwa 3,5 Gew.-%, und anschließend in einem von außen beheizten Rührbehälter bei etwa 130 bis 150°C solange gerührt, bis kein NMP-Dampf mehr abgezogen werden kann. Dabei verbleiben im Rührbehälter pastöse bis feste Ablagerungen aus einem Rückstand, der etwa 65 Gew.-% Feststoff, insbesondere Polymere des Acetylens, und noch etwa 35 Gew.-% NMP, enthält.

Nach bekannten Verfahren wird nach Beendigung des Abdampfens von NMP das Vakuum mit Stickstoff gebrochen und anschließend kaltes Wasser bei Normaldruck in den Rührbehälter eingefüllt. Das Wasser extrahiert NMP aus den Ablagerungen und wird der Abwasseraufbereitung zugeführt. Im Apparat verbleibt ein fester Rückstand, der manuell abgetragen und anschließend der Verbrennung zugeführt werden muss.

Dieses Verfahren hat wegen der Notwendigkeit der manuellen Abtragung der Ablagerungen arbeitshygienische Nachteile. Darüber hinaus entstehen Kosten für die Entsorgung der anfallenden Feststoffe.

In alternativen Verfahren wird das Problem des Feststoffhandlings vermieden, indem das verunreinigte NMP nur soweit aufkonzentriert wird, dass keine festen Ablagerungen auftreten. Derartige Verfahren haben jedoch den Nachteil, dass ein geringerer Anteil an gereinigtem NMP zurückgewonnen werden kann.

Es war demgegenüber Aufgabe der Erfindung, die bestehenden Verfahren zur Rückgewinnung von NMP, das in der Extraktion von Acetylen eingesetzt worden war, zu verbessern, insbesondere das Feststoffuandling zu vermeiden und dabei gleichzeitig einen hohen Anteil an gereinigtem NMP zurückzugewinnen.

Diese Aufgabe wird gelöst durch ein Verfahren zur Reinigung eines Apparates von Ablagerungen aus einem Verfahren zur Rückgewinnung von gereinigtem N-Methylpyrrolidon (NMP) durch Abdampfen von NMP aus einem verunreinigten NMP-Strom, der in einem Verfahren zur extraktiven Abtrennung von Acetylen aus dem Reaktionsgemisch eine Partialoxidation von Kohlenwasserstoffen nach Ausgasen des Acetylens anfällt, das dadurch gekennzeichnet ist, dass in den Apparat heißes Wasser eingeleitet und gerührt wird.

Es wurde überraschend gefunden, dass sich die pastösen bis festen Ablagerungen im Rührkessel, aus dem NMP abgedampft wurde, in einfacher Weise, mit heißem Wasser lösen lassen. Besonders vorteilhaft ist, dass das Verfahren unabhängig von der Konsistenz der Ablagerungen ist, die wiederum stark von pH-Wert abhängt, der im System stark schwankt.

Es ist möglich, den verunreinigten NMP-Strom aus der extraktiven Abtrennung von Acetylen unmittelbar in den Rührkessel einzuleiten. Vorteilhaft wird dieser jedoch zunächst im Vakuum in einer oder mehreren Stufen vorverdampft, vorzugsweise bis auf einen Polymergehalt von etwa 3,5 Gew.-%.

Als heißes Wasser wird vorwiegend Wasser bei einer Temperatur von 40 bis 180°C, bevorzugt von 50 bis 150°C, bezeichnet. Besonders bevorzugt ist Wasser mit einer Temperatur von 90°C.

Bezüglich der Wasserqualität gibt es grundsätzlich keine Einschränkungen; aus Kostengründen wird Wasser minderer Qualität bevorzugt.

Die Verweilzeit des Wassers im Apparat unter Rühren sollte mindestens 5 min betragen und liegt vorteilhaft im Bereich zwischen 5 min und 1 h, besonders bevorzugt bei ca. einer halben Stunde.

Im Apparat wird NMP solange abgedampft, bis praktisch kein Dampfdruck mehr gemessen wird. Unter diesen Voraussetzungen verbleiben im Apparat Ablagerungen aus einem pastöser bis fester Rückstand, der noch etwa 35 Gew.-% NMP, Rest polymere Feststoffe, enthält. Diese Ablagerungen bilden sich häufig in einer Schichtdicke zwischen 0,1 und 30 cm, insbesondere zwischen 1 und 10 cm, aus.

Das Volumen des in den Apparat eingeleiteten Wassers liegt vorteilhaft im Bereich von etwa 2 bis 30 mal dem Volumen der Ablagerungen, bevorzugt bei etwa 15 mal dem Volumen der Ablagerungen.

Aus dem Apparat wird eine Lösung abgezogen, die ein Papierfilter rückstandsfrei passiert, und auch beim Abkühlen stabil bleibt, ohne dass Polymer ausfällt. Die abgezogene wässrige Lösung enthält häufig ca. 2 Gew.-% Material der Ablagerungen und 2 Gew.-% NMP und hat eine wasserähnliche Viskosität im Bereich zwischen 1 und 5 mPa.s.

Der Apparat, in dem NMP zurückgewonnen und der anschließend von Verunreinigungen befreit wird, ist bevorzugt ein Rührkessel.

Das Verfahren hat somit den Vorteil, dass verunreinigtes NMP aus der extraktiven Abtrennung von Acetylen weitgehend aufkonzentriert werden kann, bis zu einem Feststoffanteil von etwa 65 Gew.-%, und einem entsprechend hohen Anteil an rückgewonnenem reinem NMP, ohne dass hierbei die Probleme mit dem Feststoffhandling, insbesondere der manuellen Entfernung der Ablagerungen sowie bei der Entsorgung, auftreten.

Die Ablagerungen aus dem Apparat werden als wässrige Lösung abgezogen, die auch nach dem Mischen mit Flusswasser zum Abkühlen auf ca. 40°C stabil bleiben, das heißt keinen Polymerausfall zeigen, ins Abwasser geleitet werden können und in der Kläranlage problemlos abgebaut werden.

Die Erfindung wird im Folgenden anhand einer Zeichnung und eines Ausführungsbeispiels näher erläutert.

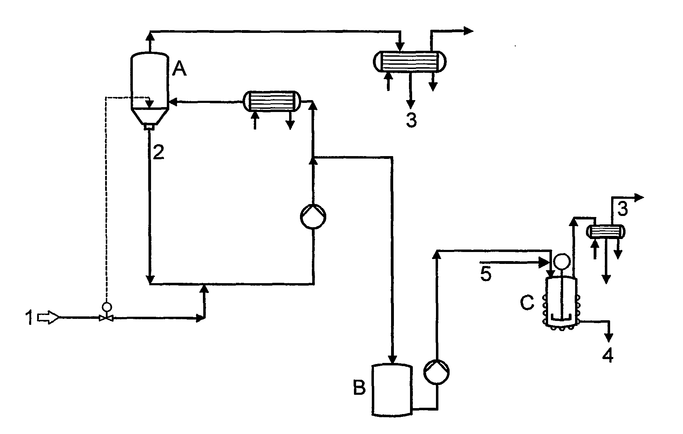

- Figur 1: zeigt das Schema einer Anlage zur Durchführung des Verfahrens zur Regenerierung von NMP, wobei auf die Modifikationen der Anlage für das erfindungsgemäße Verfahren gegenüber der Anlage aus dem Stand der Technik in der Figurenbeschreibung hingewiesen wird.

Strom 1, verunreinigtes NMP aus der extraktiven Abtrennung von Acetylen wird in einem Vorverdampfer A unter Vakuum aufkonzentriert, in einem Vorlagebehälter B gesammelt und anschließend einem Rührbehälter C mit außen liegender Begleitbeheizung zugeführt. Aus dem Rührbehälter C wird gereinigtes NMP, Strom 3, dampfförmig abgezogen und es verbleibt ein Rückstand 4 aus pastösen bis festen Ablagerungen.

Aus dem Vorverdampfer A wird ebenfalls gereinigtes NMP, Strom 3, abgezogen.

Im Verfahren nach dem Stand der Technik wird der Rückstand 4 aus dem Rührbehälter C als Feststoff manuell abgetragen.

Demgegenüber wird im erfindungsgemäßen Verfahren in den Rührbehälter C heißes Wasser Strom 5, eingeleitet, das den Rückstand unter Rühren auflöst und als wässrige Lösung abgelassen wird.

### Vergleichsbeispiel

Ein Strom von verunreinigtem NMP aus der extraktiven Abtrennung von Acetylen aus dem Reaktionsgemisch einer Partialoxidation von Kohlenwasserstoffen, mit 0,1 Gew.-% Verunreinigungen, insbesondere Oligomere und Polymere des Acetylens, wurde als Strom 1 einer Anlage zugeführt, wie sie in Figur 1 schematisch dargestellt ist. Strom 1 werde im Vorverdampfer A bei einem Vakuum von etwa 200 mbar auf einen Feststoffanteil von etwa 3,5 Gew.-% aufkonzentriert, der als Strom 2 abgezogen wurde. Über Kopf wurde gereinigtes NMP, Strom 3 abgezogen und vorzugsweise in die extraktive Abtrennung von Acetylen recycliert.

Strom 2 wurde in einem Vorlagebehälter mit der Kapazität von 3 m³ gelagert und hieraus diskontinuierlich einem Rührkessel C zugeführt, der mit einer außen liegenden Begleitbeheizung mit 4 bar Dampf auf etwa 130 bis 150°C Innentemperatur aufgeheizt wurde. Es wurde ein zylindrischer Rührkessel mit folgender Geometrie eingesetzt: Kesseldurchmesser 2 m, Kesselhöhe 2,6 m und Inhalt 4 m³. Der Rührkessel C war mit einem waagerechten Rührerblatt gerührt, das mit geringem Abstand vom Boden des Rührkessels, von etwa 2 mm, gelagert war. Aus dem Rührbehälter C wurde dampfförmig gereinigtes NMP (ebenfalls Strom 3) aus dem oberen Bereich des Rührkessels C abgezogen, solange, bis im Rührkessel C kein Dampfdruck mehr gemessen wurde.

Das Vakuum im Rührkessel C wurde mit Stickstoff gebrochen und anschließend wurde zum Abkühlen kaltes Wasser eingeleitet und nach 1 h abgelassen. Im Rührkessel C verblieben Polymerbrocken, die manuell abgetragen und zur Verbrennung zuleitet werden mussten.

Die Elementar- und Heizwertanalyse der Ablagerungen, die als kohleähnliches Polymer bezeichnet werden können, hat zu folgenden Ergebnissen geführt:

| | |
|---|---|
| Heizwert | 24.870 kJ/kg |
| Schwefel | 1 Gew.-%, |
| Wasserstoff | 6 Gew.-% und |
| Stickstoff | 4,2 Gew.-%. |

### Ausführungsbeispiel (erfindungsgemäß)

Das erfindungsgemäße Verfahren zur Rückgewinnung von NMP aus dem verunreinigten NMP-Strom aus der extraktiven Abtrennung von Acetylen unterscheidet sich bis zur Aufkonzentrierung im Rührkessel C bis kein Dampfdruck mehr gemessen werden kann, nicht von dem im Vergleichsbeispiel beschriebenen Verfahren nach dem Stand der Technik.

Die Entfernung der Ablagerungen aus dem Rührkessel C nach der Aufkonzentrierung des NMP-Stromes auf einen NMP-Restgehalt von ca. 35 Gew.-% erfolgte jedoch, abweichend vom Verfahren aus dem Vergleichsbeispiel, wie folgt: In den Rührkessel C wurden ca. 2 m³ 90° heißes Wasser auf die 5 cm hohen Ablagerungen im Rührkessel C eingeleitet und etwa eine halbe Stunde lang gerührt. Dabei lösten sich die Ablagerungen und es wurde vom Boden des Rührkessels C eine wässrige Lösung, enthaltend ca. 2 Gew.-% Material der Ablagerungen und ca. 2 Gew.-% NMP, mit einer wasserähnlichen Viskosität im Bereich zwischen 1 und 5 mPa.s, abgezogen.

Diese Lösung wurde in das behandlungsbedürftige Abwasser gegeben und in der Kläranlage problemlos abgebaut.

In der praktischen Durchführung war der Rührbehälter C auch nach 20 Chargen entsprechend dem obigen Ausführungsbeispiel frei von Ablagerungen.

## Patentansprüche

1. Verfahren zur Reinigung eines Apparates (C) von Ablagerungen aus einem Verfahren zur Rückgewinnung von gereinigtem N-Methylpyrrolidon (NMP) (3) durch Abdampfen von NMP aus einem verunreinigten NMP-Strom (1), der in einem Verfahren zur extraktiven Abtrennung von Acetylen aus dem Reaktionsgemisch einer Partialoxidation von Kohlenwasserstoffen nach Ausgasen des Acetylens anfällt, **dadurch gekennzeichnet, dass** in den Apparat (C) heißes Wasser (5) eingeleitet und gerührt wird.

2. Verfahren nach Anspruch 1, **dadurch gekennzeichnet, dass** der verunreinigte NMP-Strom (1) aus dem Verfahren zur extraktiven Abtrennung von Acetylen vor der Zuführung in den Apparat (C) in eine oder mehrere Vorverdampfungsstufen (A) geleitet wird.

3. Verfahren nach Anspruch 1 oder 2, **dadurch gekennzeichnet, dass** das heiße Wasser eine Temperatur im Bereich von 40 bis 180°C, bevorzugt von 50 bis 150°C, besonders bevorzugt eine Temperatur von etwa 90°C, aufweist.

4. Verfahren nach einem der Ansprüche 1 bis 3, **dadurch gekennzeichnet, dass** mindestens 5 min lang, bevorzugt zwischen 5 min bis 1 h, besonders bevorzugt etwa eine halbe Stunde lang, gerührt wird.

5. Verfahren nach einem der Ansprüche 1 bis 4, **gekennzeichnet durch** eine Schichtdicke der Ablagerungen zwischen 0,1 und 30 cm, insbesondere zwischen 1 und 10 cm.

6. Verfahren nach einem der Ansprüche 1 bis 5, **gekennzeichnet durch** einen Restgehalt an NMP in den Ablagerungen von etwa 35 Gew.-%.

7. Verfahren nach einem der Ansprüche 1 bis 6, **dadurch gekennzeichnet, dass** das Volumen des in den Apparat (C) eingeleiteten Wassers etwa das zwei- bis 30-fache des Volumens der Ablagerungen, bevorzugt etwa das 15-fache des Volumens der Ablagerungen, beträgt.

8. Verfahren nach einem der Ansprüche 1 bis 7, **dadurch gekennzeichnet, dass** aus dem Apparat (C) eine wässrige Lösung abgezogen wird, enthaltend etwa 2 Gew.-% Material der Ablagerungen und etwa 2 Gew.-% NMP und dass die wässrige Lösung eine Viskosität zwischen 1 und 5 mPa.s aufweist.

9. Verfahren nach einem der Ansprüche 1 bis 8, **dadurch gekennzeichnet, dass** der Apparat (C) ein Rührkessel ist.

## Claims

1. A process for cleaning an apparatus (C) to remove deposits from a process for the recovery of purified N-methylpyrrolidone (NMP) (3) by evaporating NMP from a contaminated NMP stream (1) which is obtained in a process for the extractive separation of acetylene from the reaction mixture of a partial oxidation of hydrocarbons after expulsion of the acetylene as a gas, wherein hot water (5) is passed into the apparatus (C) and is stirred.

2. The process according to claim 1, wherein the contaminated NMP stream (1) from the process for the extractive separation of acetylene is passed into one or more preevaporation stages (A) before being fed into the apparatus (C).

3. The process according to claim 1 or 2, wherein the hot water is at a temperature in the range from 40 to 180°C, preferably from 50 to 150°C, particularly preferably at a temperature of about 90°C.

4. The process according to any of claims 1 to 3, wherein stirring is effected for at least 5 min, preferably from 5 min to 1 h, particularly preferably about half an hour.

5. The process according to any of claims 1 to 4, comprising a layer thickness of the deposits of from 0.1 to 30 cm, in particular from 1 to 10 cm.

6. The process according to any of claims 1 to 5, comprising a residual NMP content in the deposits of about 35% by weight.

7. The process according to any of claims 1 to 6, wherein the volume of the water passed into the apparatus (C) is from about two to 30 times the volume of the deposits, preferably about 15 times the volume of the deposits.

8. The process according to any of claims 1 to 7, wherein an aqueous solution comprising about 2% by weight of material of the deposits and about 2% by weight of NMP is taken off from the apparatus (C), and wherein the aqueous solution has a viscosity of from 1 to 5 mPa.s.

9. The process according to any of claims 1 to 8, wherein the apparatus (C) is a stirred kettle.

## Revendications

1. Procédé pour nettoyer un appareil (C) des dépôts provenant d'un procédé de récupération de N-méthylpyrrolidone (NMP) purifiée (3) via une étape d'évaporation de la NMP à partir d'un courant de NMP contaminé (1), qui se forme après dégagement de gaz d'acétylène dans le cadre d'un procédé de séparation d'acétylène, par extraction, du mélange réactionnel d'une oxydation partielle d'hydrocarbures, **caractérisé en ce que** l'on introduit et agite de l'eau chaude (5) dans l'appareil (C).

2. Procédé selon la revendication 1, **caractérisé en ce que** le courant de NMP contaminé (1), généré par le procédé de séparation d'acétylène par extraction, est délivré, avant d'être acheminé dans l'appareil (C), à une ou plusieurs étapes de pré-évaporation (A).

3. Procédé selon la revendication 1 ou 2, **caractérisé en ce que** l'eau chaude a une température située dans la plage de 40 à 180°C, de préférence de 50 à 150 °C, mieux encore une température d'environ 90°C.

4. Procédé selon l'une quelconque des revendications 1 à 3, **caractérisé en ce que** l'on procède à une agitation sur une période d'au moins 5 mn, de préférence sur une période de 5 mn à 1 h, mieux encore sur une période d'environ une demi-heure.

5. Procédé selon l'une quelconque des revendications 1 à 4, **caractérisé en ce que** l'épaisseur de couche des dépôts se situe entre 0,1 et 30 cm, en particulier entre 1 et 10 cm.

6. Procédé selon l'une quelconque des revendications 1 à 5, **caractérisé en ce que** la teneur résiduelle en NMP dans les dépôts représente environ 35 % en poids.

7. Procédé selon l'une quelconque des revendications 1 à 6, **caractérisé en ce que** le volume d'eau introduit dans l'appareil (C) représente environ 2 à 30 fois le volume des dépôts, de préférence environ 15 fois le volume des dépôts.

8. Procédé selon l'une quelconque des revendications 1 à 7, **caractérisé en ce que** l'on extrait de l'appareil (C) une solution aqueuse contenant environ 2 % en poids de matériau des dépôts et environ 2 % en poids de NMP, et **en ce que** la solution aqueuse présente une viscosité comprise entre 1 et 5 mPa.s.

9. Procédé selon l'une quelconque des revendications 1 à 8, **caractérisé en ce que** l'appareil (C) est une cuve d'agitation.
